# EUROPEAN PATENT APPLICATION

(11) **EP 3 973 945 A1**
(43) Date of publication of application: **30.03.2022**
(21) Application number: 20198411.9
(22) Date of filing: 25.09.2020
(51) Int. Cl.: A61K 8/36, A61K 8/73, A61K 8/898, A61Q 5/06

(54) **COMPOSITION, PROCESS AND KIT FOR SEMI-PERMANENT STRAIGHTENING KERATIN FIBERS**

(71) Applicant: Kao Germany GmbH, 64297 Darmstadt (DE)
(72) Inventor: Baar, Moritz, Cincinnati, Ohio 45214-1729 (US)
(74) Representative: Grit, Mustafa

(57) **Abstract**

The present invention is on a composition, process and a kit for semi-permanent straightening hair wherein hair is applied an aqueous composition comprising an α keto carboxylic acid, thickening natural polymer and oil and/or fatty substances. The compositions shows improved semi-permanent straightening and stability.

## Description

The present invention is on a composition, process and a kit for semi-permanent straightening hair wherein hair is applied an aqueous composition comprising an α keto carboxylic acid, thickening natural polymer and oil and/or fatty substances.

Changing ones hair shape has always been in vogue. Already in the ancient Egyptian era, long, straight hair was popular. Women, which were not blessed with this kind of natural hair already used in fire heated iron plates to get their hair in the desired shape. This procedure often led to severe burns to the face and hands. It was not before the early 19th century that straightening irons became more professional with the invention of a hair tong made by Marcel Grateau. Nowadays, hot tools like straightening irons as well as blow dryers are more popular than ever before. They got into the focus of consumers in both salon and mass market. These tools lead to satisfying results as they work at high temperatures (up to 230°C for a straightening iron). When dry hair is getting in contact with the hot iron and a proper pressure is applied at the same time, the hair shape can be fixed for a certain amount of time until humidity is coming back. Unfortunately, application of such hot temperature leads to hair damage and thus a bad hair feel after their usage. Consequently, there is a huge need of products, which allow straightening of hair more effectively to limit the contact time between iron and hair while reducing damage and leaving a pleasant hair feel after application at the same time. Additionally, there is a need to completely abandon straight irons from the daily styling routine and to achieve efficiently a smooth and even result with reduced volume by only blow-drying the hair. Glyoxylic Acid is a known ingredient for hair cosmetics which allows long-lasting curl relaxation and volume reduction esp. in combination with straightening irons at high temperatures. Combined with only blow-drying of the hair, the volume reduction and esp. curl relaxation is way less effective with a lower durability and often times higher amounts of Glyoxylic Acid are needed. For both applications, there is a general need to reduce the usage amount of Glyoxylic Acid to avoid unwanted side effects. This includes a sensitizing potential as well as a stiff hair feel and an unpleasant smell that comes with the application of higher amounts of Glyoxylic Acid.

To achieve the best results with as little Glyoxylic Acid as possible is of advantage not to rinse out the product before blow-drying and optional straightening the hair. To enable an easy handling during blow drying and potential straight ironing, the dry residue needs to be as little as possible. Additionally high amounts of fatty phase (esp. fatty alcohols) need to be avoided, but still sufficient amounts of silicon oils as well as potential cationic conditioners for improved handling must be stabilized. Additionally, storage stability must be guaranteed even under low pH and with a high amount of salt.

Common solutions are emulsions to achieve a thick formulation which can be easily applied to hair without dropping or dripping. To achieve a stable and thick emulsion high amount of fatty phase, especially fatty alcohols, and emulsifier need to be used. However, during blow drying and potential straight ironing, these ingredients result in higher smoke generation as well as a sticky, greasy hair feel which results in a more difficult handling.

Another solution is to use thickener for a gel type formulation. However, many commonly used thickeners such as hydroxyethyl cellulose are not stable under low pH conditions. Anionic xantha gum, on the other hand, provides low pH stability, but is not compatible with a lot of cationic ingredients. Addtionally, xantham gum does not provide satisfactory stabilizing effects for oils such as silicone oils, mineral oils and synthetic oils, which are needed for an improved handling of hair. Importantly, xanthan gum changes its rheology during storage under above mentioned conditions, resulting in an unpleasant texture and feel.

To reduce disadvantages of both systems, it is known to separate the Glyoxylic Acid in a gel type composition and mix this prior to application with an emulsion type composition which contains cationic conditioners as well as oily components as proposed in the patent application WO 2014/131469 by Kao. This produces confusion among users and steadily ready to use compositions have been required.

In this respect, WO 2011/104282 describes a process for semi-permanent hair straightening, which involves applying a composition comprising an α-keto acid onto the hair, leaving the composition in contact with the hair for 15 to 120 minutes, drying the hair and straightening the hair with a straightening iron at a temperature of 200 +-50°C.

Furthermore, WO 2012/010351 describes a treatment for semi-permanent straightening of curly, frizzy or wavy hair by applying a solution of glyoxylic acid in combination with mechanical straightening, using a straightening iron at a temperature of 200 +- 30°C. After the treatment, the hair is said to retain its shape for at least six consecutive washings

WO 2014/072645A1 is a post-published document according to Article 54(3) EPC, which relates to a hair straightening procedure involving the application of a glyoxylic acid-containing composition onto the hair, followed by the application of a composition comprising a fatty alcohol or a cationic conditioning agent and the use heat, e.g., by means of a straightening iron. The cationic conditioning agent may be a cationic polymer. The glyoxylic acid-containing compositions may also include a nonionic cellulose-based polymer such as methylcellulose or hydroxyethylcellulose.

WO 2014/072479A2 is a post-published document according to Article 54(3) EPC and concerns a hair straightening composition comprising a certain dicarbonyl compound such as glyoxylic acid in combination with at least one cationic and/or at least one amphoteric polymer. The described compositions also may comprise hydroxypropyl methyl cellulose.

The aim of the present invention is to provide a stable composition, which does not require any further preparation at the time of use and enables efficient straightening while providing an easy handling when blow-drying and / or straight ironing due to a less sticky hair feel.

The inventor of the present invention has unexpectedly found out that an aqueous composition comprising an α keto carboxylic acid, thickening natural polymer and oil and/or fatty substances has good stability during long term storage and provides good straightening and volume reduction effects.

The first object of the present invention is an aqueous composition for semi-permanent straightening keratin fibers, especially human hair, comprising at least 0.5% by weight, of an organic acid according to the general structure

R₁-CO-COOH

wherein R₁ is selected from hydrogen, COOH, CN, optionally substituted C1-C10 alkyl, optionally substituted C2-C10 alkenyl, optionally substituted C2-C10 alkynyl, optionally substituted C3-C10 cycloalkyl, optionally substituted C6-C10 aryl or a 5-10-membered, optionally substituted heteroaryl group, wherein the optional substituents of the alkyl group are selected from halogen, hydroxyl, amino and C1-C4 alkoxy, and the optional substituents of the other groups are selected from halogen, hydroxyl, amino, C1-C4 alkyl and C1-C4 alkoxy,
at least 0.05% by weight of sclerotium gum,
at least 0.1% by weight of an oil and/or a fatty a substance, and
has a pH 4.0 or below, wherein all concentrations are calculated to the total of the composition.

The second object is a process for semi-permanent straightening keratin fibers, especially human hair, comprising the following steps performed in this order:
(a) application of the composition of the present invention onto the keratin fibes, especially human hair, preferably in a weight ratio of hair to composition of 0.5:2 to 2:0.5;
(b) leaving the composition on the hair for 5 to 120 minutes at a temperature in the range of 20 to 50°C;
(c) optionally rinsing off the composition;
(d) drying the hair with a blow dryer;
(e) treating the hair with an iron having a surface temperature of 180 +- 50°C, preferably 170 to 200°C, and
(f) optionally rinsing off and/or shampooing the hair and drying.

The third object is kit for keratin fibers especially human hair comprising the composition of the present invention.

The aqueous composition of the present invention comprises at least 0.5% by weight, of an organic acid according to the general structure given above as the active component for achieving the semi-permanent straightening effect.

Non-limiting and preferred examples are glyoxylic acid, pyruvic acid and 2-ketobutyric acid.

The organic carboxylic acid of the above structure may be comprised in the composition in its free acid form. The carbonyl group adjacent to the acid group of the acid may also be present in the hydrate form. Apart from the free acid form and the hydrate thereof, salts of the acid or the hydrate may also be used.

The hydrate of the acid of the above structure may be formed when providing the composition in an aqueous medium. For instance, glyoxylic acid (H-CO-COOH) in aqueous solution is almost quantitatively present as the hydrate (H-C(OH)₂-COOH) Besides, the hydrate may also condense to dimers. A salt of the carboxylic acid may also be used. As examples, alkali metal salts such as the sodium or potassium salt, alkaline earth metal salts such as the magnesium salt or the calcium salt and tertiary and quaternary ammonium salts may be mentioned.

In the present invention, glyoxylic acid, its salts and its hydrated form are the more preferred carboxylic acids of the above general structure.

The concentration of the at least one carboxylic acid of the above general structure and/or a hydrate thereof and/or salts thereof is in the range of 0.5 to 40%, preferably 0.75 to 40%, more preferably 1 to 35%, most preferably 2.0 to 30%, by weight, calculated to the total of the composition.

Conventional permanent hair shaping/straightening techniques are based on the reorganization of the disulfide bridges and involve a cleavage of the disulfide bonds either by using a sulfur-based reducing agent or an alkali agent, followed by the shaping of the hair and the formation of new bonds (i.e., disulfide bonds formed by the action of an oxidizing agent or thioether bonds, respectively). In contrast to these permanent straightening methods, the present invention does not utilize cleavage of the disulfide bonds and fixing the bonds in the new shape. Therefore, the semi-permanent straightening composition of the present invention does not require the presence of sulfur-based reducing agents, and preferably is free of sulfur based reducing agents. However, up to 2% by weight calculated to the total of the composition of sulfur based reducing agents does not disturb the straightening performance of the composition. Therefore, the composition usually has less than 30 2% by weight of sulfur-based reducing agents, and preferably is free of sulfur-based reducing agents.

pH of the aqueous composition is 4.0 or less, preferably in the range of 1 to 3.5, more preferably 1 to 3 and more preferably 1.5 to 3, as measured directly and at ambient temperature (25°C). The pH of the composition may be adjusted using known alkaline solutions, preferably with sodium hydroxide solution.

The aqueous composition comprises at least 0.05% by weight, sclerotium gum, preferably in the range of 0.1 to 5% more preferably 0.2 to 3% and most preferably 0.25 to % by weight, calculated to the total of the composition. Sclerotium gum is available from various suppliers such as BASF with the trade name Tinocare GL.

The aqueous composition comprises at least 0.1% by weight, preferably at a total concentration in the range of 0.2 to 30% by weight, more preferably 0.5 to 25% and most preferably 1 to 20% by weight, calculated to the total of the composition, an oil and/or a fatty substance.

The composition comprises an oil, preferably selected from silicone oils, natural oils, mineral oil and synthetic oils, and mixtures thereof.

The suitable silicone oils are dimethylpolysiloxane, and modified silicone (for example, amino-modified silicone, fluorine-modified silicone, alcohol-modified silicone, polyether-modified silicone, epoxy-modified silicone, polyoxazoline silicone (as described in JP A 2-276824), or alkyl-modified silicone), but dimethylpolysiloxane, polyether-modified silicone and aminomodified silicone are preferred.

The dimethylpolysiloxane may be any cyclic or non-cyclic dimethylsiloxane polymer, and examples thereof include SH200 series, BY22-019, BY22-020, BYII-026, B22-029, BY22-034, BY22-050A, BY22-055, BY22-060, BY22-083, FZ-4188 (all by Dow Corning Toray Co., Ltd.), KF-9008, KM-900 series, MK-15H, and MK-88 (all by Shin-Etsu Chemical Co., Ltd.).

The polyether-modified silicone may be any silicone having a polyoxyalkylene group, and the group constituting the polyoxyalkylene group may be an oxyethylene group or an oxypropylene group. More specific examples include KF-6015, KF-945A, KF-6005, KF-6009, KF-6013, KF-6019, KF-6029, KF- 6017, KF-6043, KF-353A, KF-354A, KF-355A (all by Shin-Etsu Chemical Co., Ltd.), FZ-2404, SS-2805, FZ-2411, FZ-2412, SH3771M, SH3772M, SH3773M, SH3775M, SH3749, SS-280X series, BY22-008 M, BYII-030, and BY25-337 (all by Dow Corning Toray Co., Ltd.) .

The amino-modified silicone may be any silicone having an amino group or an ammonium group, and examples thereof include an amino-modified silicone oil having all or a part of the terminal hydroxyl groups capped with a methyl group or the like, and an amodimethicone which does not have the terminals capped. A preferred example of the amino-modified silicone may be a compound represented by the following formula: 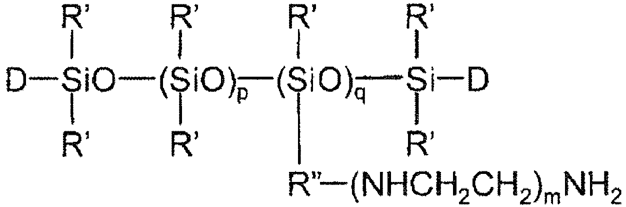 wherein R' represents a hydroxyl group, a hydrogen atom or Rx; Rx represents a substituted or unsubstituted monovalent hydrocarbon group having 1 to 20 carbon atoms; D represents Rx, R"- (NHCH2CH2)mNH2 , ORx, or a hydroxyl group; R" represents a divalent hydrocarbon group having 1 to 8 carbon atoms; m represents a number from Oto 3; p and q represent numbers, the sum of which is, as a number average, equal to or greater than 10 and less than 20,000, preferably equal to or greater than 20 and less than 3000, more preferably equal to or greater than 30 and less than 1000, and even more preferably equal to or greater than 40 and less than 800.

Specific examples of suitable commercially available products of the amino-modified silicone include amino-modified silicone oils such as SF8452C, SS-3551 (all by Dow Corning Toray Co., Ltd.), KF-8004, KF-867S, and KF-8015 (all by ShinEtsu Chemical Co., Ltd.); and amodimethicone emulsions such as SM8704C, SM8904, BY22-079, FZ-4671, and FZ-4672 (all by Dow corning Toray Co., Ltd.).

Further oil suitable oil components are hydrocarbon oils having at least 10 carbon atoms, a polyolefin, a fatty acid ester, a fatty acid amide, a polyalkylene glycol, and mixtures thereof. Examples of the hydrocarbon oil include a cyclic hydrocarbon, a linear aliphatic hydrocarbon (saturated or unsaturated), and a branched aliphatic hydrocarbon (saturated or unsaturated), and polymers or mixtures thereof are also included. The linear hydrocarbon oil preferably has 12 to 19 carbon atoms. The branched hydrocarbon oil includes hydrocarbon polymers, and preferably has more than 19 carbon atoms.

The polyolefin is a liquid polyolefin, more preferably a liquid poly-a-olefin, and even more preferably a hydrogenated liquid poly-a-olefin. The polyolefin used herein is prepared by polymerizing an olefin monomer having 4 to 14 carbon atoms, and preferably 6 to 12 carbon atoms.

The fatty acid ester may be, for example, a fatty acid ester having at least 10 carbon atoms. Examples of such a fatty acid ester include esters having a hydrocarbon chain derived from a fatty acid and an alcohol (for example, monoesters, polyhydric alcohol esters, or di- and tricarboxylic acid esters). The hydrocarbon group of these fatty acid esters may have another compatible functional group such as an amide group or an alkoxy group as a substituent, or the hydrocarbon group may be covalently bonded to those functional groups.

More specifically, an alkyl and alkenyl ester of a fatty acid having a fatty acid chain having 10 to 22 carbon atoms, a carboxylic acid ester of an aliphatic alcohol having an aliphatic chain derived from an alkyl and/or alkenyl alcohol having 10 to 22 carbon atoms, and a mixture thereof are suitably used. Specific examples of these preferred fatty acid esters include isopropyl isostearate, hexyl laurate, isohexyl laurate, isohexyl palrnitate, isopropyl palmitate, isopropyl rnyristate, decyl oleate, isodecyl oleate, hexadecyl stearate, decyl stearate, dihexadecyl adipate, lauryl lactate, myristyl lactate, cetyl lactate, oleyl stearate, oleyl oleate, oleyl myristate, lauryl acetate, cetyl propionate and dioleyl adipate.

Further suitable oil components are natural oils such as paraffin oil and natural triglycerides. Suitable natural triglycerides are argan oil, shea butter oil, karite oil, olive oil, almond oil, avocado oil, ricinus oil, coconut oil, palm oil, sesame oil, peanut oil, sunflower oil, peach kernel oil, wheat germ oil, macadamia nut oil, macadamia oil, night primrose oil, jojoba oil, castor oil, soya oil, lanolin, passiflora oil, black cumin oil, borage oils, grapeseed oil, hempseed oil, kukui nut oil, and rosehip oil.

The suitable fatty substances are such as fatty alcohols having an alkyl chain which may be saturated or unsaturated, straight or branched and with 8 to 22 C atoms. Suitable non-limiting examples are cetyl alcohol, stearyl alcohol, behenyl alcohol, and mixtures thereof.

The aqueous composition may further comprise one or more additional polymeric thickeners at a concentration lower than the concentration of the main thickener sclerotium gum. Preferably, the concentration should not exceed 50% of the main thickener, more preferably 30% by weight of the total polymeric thickeners. The additional thickener may be selected from non-ionic, anionic, cationic and amphoteric polymers having a viscosity of at least 500 mPa·s measured at a polymer concentration of 1% by weight in water and at 20°C with a Brookfield viscometer, e.g., at 10 rpm for 1 minute with an appropriate spindle.

Suitable non-limiting non-ionic polymers are include neutral polysaccharides and derivatives such as ethers or esters thereof. In this respect, neutral gums such as guar gum, hydroxypropyl guar, cellulose ethers such as hydroxyethylcellulose (HEC), methyl hydroxyethylcellulose (MHEC), ethyl hydroxyethylcellulose (EHEC), methyl ethyl hydroxyethylcellulose (MEHEC), hydroxypropylcellulose (HPC), hydroxypropylmethylcellulose (HPMC), hydrophobically modified derivatives thereof such as HM-EHEC, starch and dextrins may be mentioned.

Non-limiting examples for the anionic polymer include anionic polysaccharides and derivatives thereof, such as alginate, pectin, hyaluronate, anionic gums such as xanthan gum, dehydroxanthan gum, hydroxypropyl xanthan gum, gum arabic, gum karaya or gum tragacanth, or anionic cellulose derivatives such as carboxymethyl cellulose (CMC). Further examples include synthetic anionic polymers such as polyacrylic acid or copolymers containing acrylic acid in combination with neutral vinylic and/or acrylic monomers, and salts thereof such as sodium polyacrylate.

Non-limiting examples of cationic polymers include cationized cellulose, cationic starch, cationic guar gum, a vinylic or (meth)acrylic polymer or copolymer having quaternary ammonium side chains, (meth)acrylate/aminoacrylate copolymer, amine substituted polyacrylate crosspolymers, a polymer or copolymer of a diallyl quaternary ammonium salt, and quaternized polyvinylpyrrolidone. As an example of the vinylic or (meth)acrylic polymer or copolymer having quaternary ammonium side chains, poly(2-methacryloxyethyltrimethylammonium chloride) (Polyquaternium- 37) may be mentioned. Specific examples of the quaternized polyvinylpyrrolidone include quaternary ammonium salts synthesized from a copolymer of vinylpyrrolidone (VP) and dimethylaminoethyl methacrylate, and diethyl sulfate (polyquaternium-11). As an example of the (meth)acrylate/aminoacrylate copolymer, Acrylates/Aminoacrylates/Cl0-30 Alkyl PEG-20 Itaconate Copolymer may be mentioned. As an example of the amine substituted polyacrylate crosspolymers, Polyacrylates-1 Crosspolymer may be mentioned. Specific examples of the cationized cellulose include a polymer of a quaternary ammonium salt obtained by adding glycidyltrimethylammonium chloride to hydroxyethylcellulose (polyquaternium 10), and a hydroxyethylcellulose/ dimethyldiallylammonium chloride copolymer (polyquaternium- 4), and a polymeric quaternary ammonium salt of hydroxyethyl cellulose reacted with a trimethyl ammonium substituted epoxide and a lauryl dimethyl ammonium substituted epoxide (polyqauternium-67).

As examples for the amphoteric polymer, carboxyl-modified or sulfonate-modified cationic polysaccharides such as carboxymethylchitosan may be mentioned. Further examples include copolymers of cationic vinylic or (meth)acrylic monomers with (meth)acrylic acid, such as dimethyldiallylammonium chloride/acrylic acid copolymer (polyquaternium-22).

The composition may be in the form of an emulsion and comprise emulsifiying surfactants selected from a cationic surfactant, a nonionic surfactant, an amphoteric surfactant and an anionic surfactant.

The cationic surfactnts amy be quaternary ammonium surfactant of the general formula 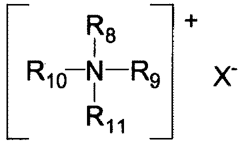
wherein R₈ and R₉ are independent from each other a saturated or unsaturated, branched or straight alkyl chain with 8-22 C atoms or

   R₁₂-CO-NH-(CH₂)ₙ
wherein R₁₂ is a saturated or unsaturated, branched or straight alkyl chain with 7-21 C atoms and n is an integer of 1 - 4, or

   R₁₂CO-O-(CH₂)ₙ
wherein R12 and n are same as above,
R10 and R11 are independent from each other an alkyl group with 1 to 4 carbon atoms, hydroxyl alkyl chain with 1 to 4 carbon atoms, or ethoxy or propoxy group with a number of ethoxy or propoxy groups varying in the range of 1 to 4, and X is chloride, bromide, methosulfate or ethosulfate.

Suitable cationic surfactants are, for example, long-chain quaternary ammonium compounds which can be used alone or in admixture with one another, such as cetyl trimethyl ammonium chloride, myristyl trimethyl ammonium chloride, behentrimonium chloride, trimethyl cetyl ammonium bromide, stearyl trimethyl ammonium chloride, stearyl trimonium chloride and stearamidopropyltrimoniurn chloride.

Suitable non-limiting examples of the nonionic surfactant include higher (C₁₂-C₂₄) fatty acid sucrose ester, polyglycerin C₈₋₂₄-fatty acid ester, higher (C₁₂-C₂₄) fatty acid mono- or diethanolamide, polyoxyethylene hardened castor oil, polyoxyethylene sorbitan (C₁₂₋₂₄) -fat ty acid ester, C₈₋₂₄-alkylamine oxide, and C₈₋₂₄-alkylamidoamine oxide, an ethoxylated fatty alcohols according to general structure

R₂-O-(CH₂CH₂O)ₙH

wherein R₂ is a saturated alkyl chain with 8 to 24 C atoms and n is a value in the range of 10 to 50.

Examples of the amphoteric surfactant include an imidazoline- based surfactant, a carbobetaine-based surfactant, an amidobetaine-based surfactant, a sulfobetaine-based surfactant, a hydroxysulfobetaine-based surfactant and an amidosulfobetaine-based surfactant.

Examples of the anionic surfactant include alkylbenzenesulfonate, alkyl or alkenyl ether sulfate, alkyl or alkenyl sulfate, olefin sulfonate, alkanesulfonate, saturated or unsaturated fatty acid salts, alkyl or alkenyl ether carboxylate, a-sulfo fatty acid salts, N-acylamino acid type surfactants, phosphoric acid mono- or diester type surfactants, and sulfosuccinate. Examples of the alkyl ether sulfate include polyoxyethylene alkyl ether sulfate. Examples of the counterion for the anionic residues of these surfactants include an alkalimetal ion such as sodium ion or potassium ion; an alkaline earth metal ion such as calcium ion or magnesium ion; an ammonium ion; and an alkanolamine having 1 to 3 alkanol groups each having 2 or 3 carbon atoms (for example, monoethanolamine, diethanolamine, triethanolamine, or triisopropanolamine).

The surfactant can be used singly or in combination of two or more kinds. When adding a surfactant to the straightening composition, the content thereof usually is 0.05 to 10% wt.%, more preferably 0.1 to 5 wt.%, based on the total weight of the straightening composition.

The compositions may comprise additional compounds found customarily in such cosmetic compositions such as preservatives, fragrances and others.

The semi-permanent straightening process is carried out comprising the following steps performed in this order:
(a) application of the aqueous composition of the present invention onto the hair, preferably in a weight ratio of hair to composition of 0.5:2 to 2:0.5;
(b) leaving the composition on the hair for 5 to 120 minutes at a temperature in the range of 20 to 50°C;
(c) optionally rinsing off the hair;
(d) drying the hair with a blow dryer;
(e) treating the hair with an iron having a surface temperature of 180 +- 50°C, preferably 170 to 200°C, and
(f) optionally rinsing off and/or shampooing the hair and drying.

In step (a) of the process of the present invention, the aqueous composition composition is applied to the hair. The application weight ratio of hair to composition is 0.5:2 to 2:0.5, preferably 0.5:1 to 1:0.5, more preferably about 1:1. Subsequent to the application, the straightening composition is left on the hair for 5 to 120 minutes, preferably 5 to 90 minutes, more preferably 10 to 60 minutes and more preferably 15 to 45 minutes at a temperature range of 20 to 50°C, preferably at ambient temperature (step (b)). Then, the composition is optionally rinsed off from hair (step (c)). In subsequent step (d), the hair is dried with a blow drier. It is preferable to dry the hair under continuous combing in order to prevent entanglement of the hair. Subsequent to the drying, the hair is treated with an iron having a surface temperature of 180±50°C, preferably 170 to 200°C. A usual straightening iron may be used for this purpose (step (e)). Furthermore, the hair may optionally be rinsed off with water and/or shampooed and dried again (step (f)).

The aqueous composition is provided to the user in a kit, which comprises the composition of the present invention and optionally one or more of additional compositions and /or hair appliances such as a hair drier. In the preferred form the kit comprises the composition of the present invention and a hair drier and optionally a hair straightening iron.

### Example

The following compositions were prepared with conventional technique and stability of both compositions were determined for 3 months under various conditions.

| | % by weight | |
|---|---|---|
| | Comparative | Inventive |
| Water | to 100 | to 100 |
| Dehydroxanthan gum | 0,6 | - |
| Sclerotium gum | - | 0,6 |
| 1,2-propylene glycol | 1 | 1 |
| C12-13-Pareth-9 | 1 | 1 |
| Polysilicone-29 | 0,8 | 0,8 |
| Glyoxylic acid | 20 | 20 |
| Sodium Hydroxide | 1 | 1 |
| Trisiloxane | 10 | 10 |
| Amodimethicone | 1,1 | 1,1 |
| Fragrance | 0,5 | 0,5 |

Stability of the compositions were measured at room temperature, 40°C and at 50°C for 3 months. The viscosity of the compositions were determined with Brookfiled viscosimeter with Spindle 4 at RPM, all measurements were carried out at 20°C. Visual inspection was carried out with naked eye.

The following stability data were observed.

### Comparative

### composition

**Freshly prepared:** Hazy, colourless and homogeneous appearance with a viscosity of 2940 mPa.s

| 1 Month | RT | 40°C | 50°C |
|---|---|---|---|
| | 3760 mPa.s | 4340 mPa.s | 2930 mPa.s |
| | Hazy, colourless and homogeneous | Hazy, slightly yellowish and homogeneous | Hazy, yellowish and separated spots on top |
| | | | |

| 3 Months | 3560 mPa.s | 3480 mPa.s | - |
|---|---|---|---|
| | Hazy, colourless and some spots on top | Hazy, yellowish and separated oil on top | - |

### Inventive composition

**Freshly prepared**: Hazy, colourless and homogeneous appearance with a viscosity of 2720 mPa.s

| 1 Month | RT | 40°C | 50°C |
|---|---|---|---|
| | 3860 mPa.s | 4580 mPa.s | 3700 mPa.s |
| | Hazy, colourless and homogeneous | Hazy, slightly yellowish and homogeneous | Hazy, slightly yellowish and homogeneous |
| | | | |

| 3 Months | 3600 mPa.s | 4620 mPa.s | 3380 mPa.s |
|---|---|---|---|
| | Hazy, colourless and some spots on top | Hazy, slightly yellowish and homogeneous | Hazy, yellowish and homogeneous |

From the above results it is beyond any doubt that the inventive compositions showed improved stability which is owing to the change of the thickening polymer from dehydroxanthan gum to sclerotium gum.

## Claims

1. An aqueous composition for straightening and/or volume reduction of keratin fibers, especially human hair, **characterized in that** it comprises at least 0.1% by weight, of an organic acid according to the general structure
R₁-CO-COOH
wherein R₁ is selected from hydrogen, COOH, CN, optionally substituted C1-C10 alkyl, optionally substituted C2-C10 alkenyl, optionally substituted C2-C10 alkynyl, optionally substituted C3-C10 cycloalkyl, optionally substituted C6-C10 aryl or a 5-10-membered, optionally substituted heteroaryl group, wherein the optional substituents of the alkyl group are selected from halogen, hydroxyl, amino and C1-C4 alkoxy, and the optional substituents of the other groups are selected from halogen, hydroxyl, amino, C1-C4 alkyl and C1-C4 alkoxy,
at least 0.05% by weight of naturally derived non-ionic gum, preferably sclerotium gum,
at least 0.1% by weight of an oil and/or fatty substance, and
has a pH 4.0 or below,
wherein all concentrations are calculated to the total of the composition.

2. The composition according to claim 1 **characterized in that** the pH of the composition is 3 or less, especially in the range of 1 to 3.

3. The composition according to claims 1 and/or 2 **characterized in that** the organic acid is glyoxylic acid or hydrates thereof or their salts.

4. The composition according to any of the preceding claims **characterized in that** the organic acid is comprised at a concentration in the range of 0.1 to 40% by weight, preferably 1.0 to 40% by weight, more preferably 2.5 to 40% by weight, in particular 2.5 to 25% by weight, based on the weight of the total composition

5. The composition according to any of the preceding claims **characterized in that** the nonionic gum, preferably sclerotium gum, is comprised at a concentration in the range of 0.1 to 5% by weight, calculated to the total of the composition.

6. The hair straightening process according to any of the claims 1 to 6, wherein the hair straightening composition is an emulsion.

7. The composition according to any of the preceding claims **characterized in that** it comprises one or more oils, preferably selected from silicone oils, natural oils, mineral oil and synthetic oils, at a total concentration in the range of 1 to 20% by weight calculated to the total composition.

8. The composition according to any of the preceding claims **characterized in that** it comprises silicone oil, preferably trisiloxane.

9. The hair straightening process according to claim 7, wherein the hair straightening composition comprises at least one fatty alcohol having 8 to 22 carbon atoms.

10. The composition according to any of the preceding claims **characterized in that** it comprises one or more surfactants selected from anionic, amphoteric, nonionic and cationic ones at a concentration in the range of 0.1 to 10% by weight, calculated to the total of the composition.

11. The composition according to any of the preceding claims **characterized in that** it comprises quaternary ammonium surfactant of the general formula 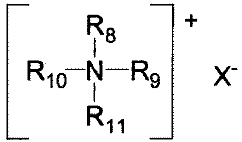
wherein R₈ and R₉ are independent from each other a saturated or unsaturated, branched or straight alkyl chain with 8-22 C atoms or R₁₂-CO-NH-(CH₂)ₙ wherein
R₁₂ is a saturated or unsaturated, branched or straight alkyl chain with 7-21 C atoms and n is an integer of 1 - 4, or R12CO-O-(CH2)n wherein
R12 is a saturated or unsaturated, branched or straight alkyl chain with 7-21 C atoms and n is an integer of 1 - 4, and
R10 and R11 are independent from each other an alkyl group with 1 to 4 carbon atoms, hydroxyl alkyl chain with 1 to 4 carbon atoms, or ethoxy or propoxy group with a number of ethoxy or propoxy groups varying in the range of 1 to 4, and X is chloride, bromide, methosulfate or ethosulfate.

12. The composition according to any of the preceding claims **characterized in that** it comprises one or more nonionic surfactants, preferably selected from fatty alcohol ethoxylates according to general structure
R₂-O-(CH₂CH₂O)ₙH
wherein R₂ is a saturated alkyl chain with 8 to 24 C atoms and n is a value in the range of 10 to 50.

13. The composition according to any of the preceding claims **characterized in that** it comprises a second thickening polymer selected from anionic, cationic, non-ionic and amphoteric polymers having a viscosity of at least 500 mPa·s measured at a polymer concentration of 1% by weight, in water and at 20°C with a Brookfield viscometer, preferably at a concentration not more than the concentration of sclerotium gum, more preferably at a concentration up to 50% of the concentration of sclerotium gum.

14. Process for semi-permanent hair straightening, comprising the following steps performed in this order:
(a) application of a hair straightening composition according to claims 1 to 13 onto the hair, preferably in a weight ratio of hair to composition of 0.5:2 to 2:0.5;
(b) leaving the composition on the hair for 5 to 120 minutes at a temperature in the range of 20 to 50°C;
(c) optionally rinsing off the hair;
(d) drying the hair;
(e) treating the hair with an iron having a surface temperature of 180 +- 50°C, preferably 170 to 200°C, and
(f) optionally rinsing off and/or shampooing the hair and drying.

15. Kit for hair comprising the composition as defined in any of the claims 1 to 13.
